# EUROPÄISCHE PATENTSCHRIFT

(11) **EP 3 287 093 B1**
(45) Veröffentlichungstag und Bekanntmachung des Hinweises auf die Patenterteilung: **27.09.2023**
(21) Anmeldenummer: 17187063.7
(22) Anmeldetag: 21.08.2017
(51) Int. Cl.: A61B 34/00, A61B 17/70, A61B 34/10, A61B 34/20, A61B 90/00, A61B 17/88, A61B 90/50, A61B 90/30

(54) **MEDIZINISCHES INSTRUMENTARIUM UND VERFAHREN**
SET OF MEDICAL INSTRUMENTS AND METHOD
INSTRUMENT MÉDICAL ET PROCÉDÉ

(30) Priorität: 23.08.2016 DE 102016115605
(43) Veröffentlichungstag der Anmeldung: 28.02.2018
(73) Patentinhaber: Aesculap AG, 78532 Tuttlingen (DE)
(72) Erfinder: Beger, Jens, 78532 Tuttlingen (DE); Kozak, Josef, 78532 Tuttlingen (DE)
(74) Vertreter: Hoeger, Stellrecht & Partner Patentanwälte mbB

(56) Entgegenhaltungen:
- EP-A1- 1 667 067
- EP-A1- 1 719 472
- EP-A1- 2 910 206
- WO-A1-2016/134904
- WO-A1-2016/134911
- DE-A1- 10 314 882
- DE-A1-102005 026 654

## Beschreibung

Die Erfindung betrifft ein medizinisches Instrumentarium.

Außerdem betrifft die Erfindung ein Verfahren zum Ermitteln der Form eines medizintechnischen Artikels.

Der medizintechnische Artikel kann hinsichtlich Form und Funktion auf unterschiedlichste Weise ausgestaltet sein. Beispielsweise ist der medizintechnische Artikel ein Implantat. Ein Beispiel für einen Artikel in Gestalt eines Implantates ist ein Stabilisierungselement eines medizinischen Fixationssystems. Bei einer unbeanspruchten Ausführungsform kann der medizintechnische Artikel ein chirurgisches Instrument sein.

Die Erfindung wird vorliegend insbesondere am Beispiel eines Implantates und speziell eines Stabilisierungselementes beschrieben.

Stabilisierungselemente kommen insbesondere als Bestandteile von chirurgischen Fixationssystemen zum Einsatz. Mittels eines derartigen Fixationssystemes lassen sich beispielsweise Knochen oder Knochenstücke relativ zueinander fixieren; ein typisches Anwendungsgebiet ist die Wirbelsäulenchirurgie, bei der Wirbelkörper gegen Bewegung relativ zueinander gesichert werden sollen. Dabei werden in den Wirbelkörpern Verankerungselemente, beispielsweise Knochenschrauben, verankert und mittels des Stabilisierungselementes, beispielsweise eines Stabes, miteinander verbunden. Ein derartiges Fixationssystem ist beispielsweise in der DE 10 2010 016 448 A1 beschrieben.

Um einen Eingriff möglichst geringer Invasivität vorzunehmen ist es wünschenswert festzustellen, ob das Stabilisierungselement dazu geeignet ist, die Verankerungselemente so miteinander zu verbinden, dass die gewünschte Fixation erzielt wird. Erforderlichenfalls kann die Form des Stabilisierungselementes geändert oder ein Stabilisierungselement aus einer Mehrzahl von zur Verfügung stehenden Stabilisierungselementen unterschiedlicher Form ausgewählt werden. Vorzugsweise erfolgt die Feststellung, die Formänderung und/oder die Auswahl vor der Implantation des Stabilisierungselementes.

In der Patentanmeldung DE 10 2015 102 776 A1 ist beschrieben, wie die Form eines Stabilisierungselementes anhand von Aufnahmen ermittelbar ist, die mittels eines Navigationssystems erstellt und von einer Datenverarbeitungseinheit verarbeitet werden. Mit einer Erfassungseinheit des Navigationssystems werden zumindest zwei Aufnahmen des Stabilisierungselementes und einer in definierter räumlicher Anordnung am Stabilisierungselement gehaltenen oder von diesem umfassten oder gebildeten Markiereinrichtung erstellt, wobei die zwei oder mehr Aufnahmen von der Datenverarbeitungseinheit ausgewertet werden.

In der WO 2016/134904 A1 ist ein medizinisches Instrumentarium mit einem handhaltbaren integrierten medizintechnischen Navigationssystem beschrieben, welches eine eine Kamera aufweisende optische Erfassungseinheit aufweist, eine Datenverarbeitungseinheit und eine optische Anzeigeeinheit. Lage- und/oder Orientierungsdaten einer medizinischen Markiereinrichtung, die mittels der Erfassungseinheit erfassbar ist, sind von der Datenverarbeitungseinheit verarbeitbar, und diesbezügliche Informationen sind an der Anzeigeeinheit darstellbar. Das Instrumentarium weist eine Beleuchtungseinheit auf, mit der Licht in Richtung der mit der Erfassungseinheit erfassbaren Markiereinrichtung aussendbar ist.

Ein medizinisches Instrumentarium mit zwei oder insbesondere mehr Verankerungselementen zum Verankern an Körpergewebe und mit einem Stabilisierungselement, über das die zwei oder mehr Verankerungselemente miteinander verbindbar sind, ist in der EP 2 910 206 A1 beschrieben. Das Instrumentarium umfasst eine Sensoreinheit, eine Kopplungseinheit zum perkutanen selektiven Koppeln der Sensoreinheit mit mindestens einem Verankerungselement oder mit dem Stabilisierungselement sowie eine Datenverarbeitungseinheit, die anhand von Sensorsignalen der Sensoreinheit die Position der Verankerungselemente relativ zueinander und/oder die Position mindestens eines Verankerungselementes relativ zum Stabilisierungselement ermittelt.

In der DE 103 14 882 A1, der US 2005/0262911 A1 und der US 8,549,888 B2 sind jeweils Vorrichtungen beschrieben, mit denen die Form chirurgischer Stabilisierungselemente verändert werden kann.

Aufgabe der vorliegenden Erfindung ist es, ein Instrumentarium und ein Verfahren bereitzustellen, mit dem die Form eines medizintechnischen Artikels auf einfache Weise ermittelt werden kann.

Diese Aufgabe wird erfindungsgemäß durch ein medizinisches Instrumentarium mit den Merkmalen von Anspruch 1 gelöst.

In die vorliegende Erfindung fließt der Gedanke mit ein, dass mittels eines medizinischen Navigationssystems eine Markiereinrichtung hinsichtlich Lage und Orientierung und dementsprechend Lageänderung und Orientierungsänderung erfasst werden kann. Die Lage und Orientierung kann dabei relativ zur Erfassungseinheit festgestellt werden, wobei die Markiereinrichtung ihrerseits ein Referenzkoordinatensystem definiert. Das Navigationssystem weist eine optische Erfassungseinheit mit mindestens einer Kamera auf, mit der Aufnahmen des medizintechnischen Artikels, jedenfalls zumindest eines Abschnittes davon, einschließlich der daran angeordneten Markiereinrichtung erstellt werden können. Die Position und die Form des Artikels kann aus genau einer Aufnahme von der Datenverarbeitungseinheit ermittelt werden. Hierzu können in der Datenverarbeitungseinheit Bildverarbeitungsalgorithmen ausführbar hinterlegt sein, die die eine Aufnahme der mindestens einen Kamera analysieren und anhand der Lage- und/oder Orientierungsinformation aufgrund der Markiereinrichtung die dreidimensionale Gestalt des Artikels in hinreichender Genauigkeit bestimmen. Die Bestimmung der Position des Artikels kann im Referenzkoordinatensystem aufgrund der definierten räumlichen Beziehung der Markiereinrichtung zum Artikel festgestellt werden.

Das erfindungsgemäße Instrumentarium erlaubt es insbesondere, die Form des Artikels zu ermitteln, ohne dass dieser zu diesem Zweck räumlich fixiert sein muss. Der apparative Aufwand, um die Form des Artikels zu ermitteln, kann dadurch gering gehalten werden. Dies erleichtert auch die Handhabung des Instrumentariums.

Günstig ist, dass in einer Speichereinheit Informationen über den Artikel gespeichert sind, die von der Datenverarbeitungseinheit bei der Ermittlung der Form des Artikels herangezogen werden. In der Speichereinheit können beispielsweise Informationen über Abmessungen des Artikels hinterlegt sein, zum Beispiel dessen Länge, Breite oder Durchmesser. Durch Vergleich von Abmessungen, die in Bezug auf unterschiedliche Artikel in der Speichereinheit hinterlegt sind, mit anhand von der einen Aufnahme ermittelten abgeschätzten Abmessungen gibt dies auch die Möglichkeit, den Artikel zu identifizieren und die Form anhand der in der Speichereinheit hinterlegten Informationen zu rekonstruieren.

Für eine konstruktiv einfache Ausgestaltung und eine kostengünstige Fertigung des Instrumentariums ist es günstig, dass das Navigationssystem ein handhaltbares integriertes Navigationssystem ist. Unter "integriert" wird vorliegend verstanden, dass die Erfassungseinheit und die Datenverarbeitungseinheit in einem gemeinsamen Gehäuse angeordnet sind. In dem Gehäuse ist vorzugsweise eine Anzeigeeinheit des Navigationssystems angeordnet.

Es kann vorgesehen sein, dass eine Mehrzahl von Aufnahmen der Markiereinrichtung und des Artikels oder zumindest eines Abschnittes davon erstellbar ist, die vorzugsweise unter unterschiedlicher Orientierung erstellt werden. Dies gibt die Möglichkeit, die Form des Artikels noch zuverlässiger zu bestimmen.

Günstig ist es, wenn aufeinanderfolgend mehr als zwei Aufnahmen des Artikels und der Markiereinrichtung erstellbar sind, anhand derer die Form des Artikels ermittelbar ist, wobei die Orientierungen und Aufnahmen paarweise voneinander unterschiedlich sind. Dadurch lässt sich die Form des Artikels noch genauer ermitteln.

Weist der Artikel eine Längserstreckung auf, beispielsweise bei stabförmiger Ausgestaltung, werden die Aufnahmen bevorzugt unter Ausrichtung einer optischen Achse der mindestens einen Kamera im Winkel und insbesondere quer zur Längsrichtung des Artikels aufgenommen. Zwischen zwei Aufnahmen wird die Kamera bevorzugt um 90° bezüglich der Längserstreckung des Artikels gedreht.

Vorteilhafterweise umfasst die Erfassungseinheit genau eine Kamera, um die konstruktive Ausgestaltung des Navigationssystems zu vereinfachen. Das Vorsehen einer Stereokamera ist nicht erforderlich.

Beispielsweise ist das handhaltbare integrierte Navigationssystem ein Smartphone oder ein Tablet-Computer. Auf dem Smartphone oder Tabletcomputer kann ein Datenverarbeitungsprogramm ausführbar gespeichert sein, mit dem Daten der Erfassungseinheit von der Datenverarbeitungseinheit analysiert und die Form des Artikels ermittelt werden können.

Bei einer vorteilhaften Ausführungsform kann das Navigationssystem eine Datenbrille sein oder umfassen. Die Datenbrille kann nach Art einer Brille oder eines Brillengestells getragen werden. Nicht erforderlich ist, dass Brillengläser, geschliffen oder ungeschliffen (Glas oder Kunststoff) vorhanden sind. Ausreichend ist beispielsweise ein Gestell zum An- oder Aufliegen an den Ohren und der Nase, an dem die optische Erfassungseinheit mit der Kamera, eine Anzeigeeinheit und vorzugsweise die Datenverarbeitungseinheit gehalten sind. Die Speichereinheit und/oder eine Beleuchtungseinheit kann ebenfalls an dem Gestell gehalten sein. An der Datenbrille kann eine Batterie zur Energieversorgung vorgesehen sein.

Von Vorteil ist es, wenn das Navigationssystem eine mit der Datenverarbeitungseinheit gekoppelte Anzeigeeinheit umfasst, an der die Aufnahmen des Artikels darstellbar oder bereitstellbar sind und/oder Hinweise für einen Benutzer, die Aufnahmen durchzuführen und/oder eine Darstellung des Artikels, ermittelt anhand der Aufnahmen.

Das Navigationssystem kann eine Beleuchtungseinheit aufweisen, mit der Licht, insbesondere sichtbares Licht, in Richtung auf die Markiereinrichtung emittierbar ist. Die Beleuchtungseinheit umfasst zum Beispiel mindestens eine LED-Lichtquelle und wird vorzugsweise von dem integrierten, handhaltbaren Navigationssystem umfasst.

Der medizintechnische Artikel kann bei einer nicht beanspruchten Ausführungsform ein chirurgisches Instrument sein.

Erfindungsgemäß ist der medizintechnische Artikel ein Implantat.

Bei dem Implantat kann es sich um ein im Körper verbleibendes Implantat handeln oder um ein nur vorübergehend verwendetes Probeimplantat, das auch als Werkzeug des Instrumentariums angesehen werden kann.

Das Instrumentarium kann ein Implantationswerkzeug für das Implantat aufweisen, an dem dieses und die Markiereinrichtung gehalten sind. Das Implantationswerkzeug ist beispielsweise handgeführt und erlaubt es, das Implantat bevorzugt perkutan und minimalinvasiv zu implantieren. Über das Implantationswerkzeug ist die Markiereinrichtung in definierter räumlicher Anordnung zum Implantat angeordnet. Die Markiereinrichtung kann mit dem Implantationswerkzeug lösbar verbindbar sein. Vorliegende Ausführungsform erlaubt es, die Form des als Implantat ausgestalteten Artikels zu ermitteln. Zugleich ist die Möglichkeit gegeben, das Implantat über das Implantationswerkzeug mit der daran gehaltenen Markiereinrichtung bei der Implantation zu verfolgen. Besonders bei perkutaner Implantation wird dadurch die Handhabung des Artikels erheblich vereinfacht.

Das Implantat ist bei der erfindungsgemäßen Ausführungsform des Instrumentariums ein Stabilisierungselement eines chirurgischen Fixationssystems. Auf derartige Fixationssysteme und Stabilisierungselemente wurde bereits eingangs eingegangen.

Das Stabilisierungselement kann insbesondere ein Stab sein.

Vorzugsweise weist das Instrumentarium eine Verformungseinrichtung auf, mit der die Form des Stabilisierungselementes veränderbar ist.

Die Verformungseinrichtung ist zum Beispiel eine Biegeeinrichtung zum Biegen eines Stabes, als der das Stabilisierungselement ausgestaltet ist.

Günstig ist es, wenn einer Bedienperson von der Datenverarbeitungseinheit an einer Hinweiseinheit des Navigationssystems, beispielsweise an einer Anzeigeeinheit, Formänderungsinformationen zur Handhabung der Verformungseinrichtung bereitstellbar sind, um das Stabilisierungselement ausgehend von der ermittelten Form in eine vorgebbare Form zu bringen. Beispielsweise kann die Datenverarbeitungseinheit vergleichen, ob die ermittelte Form des Stabilisierungselementes mit einer erforderlichen, gewünschten Form übereinstimmt. Die erforderliche Form kann zum Beispiel ermittelt werden, indem die Relativpositionen von Verankerungselementen des Fixationssystems festgestellt werden mit der Maßgabe, dass die Verankerungselemente mit dem Stabilisierungselement zu verbinden sind. Weicht die anhand der Aufnahmen ermittelte Form von der erforderlichen Form ab, kann das Stabilisierungselement mit der Verformungseinrichtung verformt werden. Zu diesem Zweck können der Bedienperson an der Anzeigeeinheit Formänderungsinformationen bereitgestellt werden und dadurch die Handhabung des Instrumentariums erheblich vereinfacht werden.

In entsprechender Weise ist es günstig, wenn vom Navigationssystem über eine Kommunikationsschnittstelle Formänderungsinformationen an die Verformungseinrichtung übertragbar sind, um das Stabilisierungselement ausgehend von der ermittelten Form in eine vorgebbare Form zu bringen. Die Verformungseinrichtung kann das Stabilisierungselement vorzugsweise ohne Zutun der Bedienperson anhand der ihr zugeführten Formänderungsinformationen in die vorgebbare, erforderliche Form bringen.

Insbesondere ist denkbar, dass die Form des Stabilisierungselementes in-situ erfasst und/oder bevorzugt in-situ mittels der Verformungseinrichtung verändert wird. Alternativ kann vorgesehen sein, dass die Form vom Stabilisierungselement ex-situ erfasst und/oder ex-situ mittels der Verformungseinrichtung verändert wird.

Wie erwähnt, kann der Artikel bei einer nicht beanspruchten Ausführungsform ein chirurgisches Instrument sein.

Bei dieser vorteilhaften Ausführungsform des Instrumentariums kann das chirurgische Instrument ein Schraubinstrument oder ein Einschlaginstrument sein. Unter Einsatz der Markiereinrichtung kann es sich bei dem Instrument dementsprechend um ein navigiertes Schraubinstrument oder ein navigiertes Einschlaginstrument handeln. Mittels des Instrumentariums ist die Möglichkeit gegeben, etwaige Verformungen des Schraubinstrumentes (beispielsweise eine Unrundheit) oder des Einschlaginstrumentes festzustellen. Die Form des Instrumentes, ermittelt anhand der einen Aufnahme, kann mit einer beispielsweise aus einer Speichereinheit gelesenen Form des Instrumentes verglichen und dadurch eine Verformung ermittelt werden. Beim Einsatz des Instrumentes können derartige Verformungen kompensiert werden. Der Benutzer kann über die Verformung des Instrumentes beispielsweise an einer Anzeigeeinheit des Instrumentariums informiert werden und die Handhabung des Instrumentes entsprechend anpassen.

Es kann vorgesehen sein, dass der Artikel Markierelemente der Markiereinrichtung umfasst oder bildet, beispielsweise sind die Markierelemente ausgestaltet als bevorzugt reflektierende Punkte oder Linien am Artikel.

Die Markiereinrichtung kann am Artikel mittelbar oder unmittelbar lösbar festgelegt oder festlegbar sein.

Bei einer vorteilhaften Ausführungsform des Instrumentariums ist die Markiereinrichtung einstückig mit dem Artikel verbunden, beispielsweise an diesen angeformt oder angeschweißt.

Günstigerweise ist eine Sollbruchstelle vorgesehen zum Trennen der Markiereinrichtung vom Artikel. Nach Ermitteln der Form des Artikels kann die Markiereinrichtung von diesem getrennt werden. Ein als Implantat und speziell Stabilisierungselement ausgestalteter Artikel kann beispielsweise anschließend implantiert werden.

Es kann vorgesehen sein, dass eine Mehrzahl von Markiereinrichtungen direkt oder indirekt am Artikel gehalten und in definierter räumlicher Anordnung zu diesem positioniert oder von diesem umfasst oder gebildet ist, wobei anhand von mehreren Aufnahmen, die jeweils eine Markiereinrichtung und einen Abschnitt des Artikels erfassen, die Position und Form des Abschnitts des Artikels von der Datenverarbeitungseinheit ermittelbar ist. Beispielsweise ist eine Mehrzahl derartiger Markiereinrichtungen am Artikel gehalten oder von diesem gebildet. Eine jeweilige Markiereinrichtung kann mit einem jeweiligen Abschnitt des Artikels erfasst und daraus die Position und die Form des Abschnittes des Artikels anhand der einen Aufnahme ermittelt werden. Dies kann vorzugsweise in-situ durchgeführt werden, jedoch ist auch eine ex-situ Erfassung und Ermittlung der Form der jeweiligen Abschnitte möglich.

Wie bereits erwähnt, betrifft die vorliegende Erfindung auch ein Verfahren. Die eingangs gestellte Aufgabe wird durch ein erfindungsgemäßes Verfahren mit den Merkmalen von Anspruch 13 gelöst.

Die Vorteile, die bereits im Zusammenhang mit der Erläuterung des erfindungsgemäßen Instrumentariums erwähnt wurden, können unter Ausübung des Verfahrens ebenfalls erzielt werden. Diesbezüglich kann auf die voranstehenden Ausführungen verwiesen werden.

Vorteilhafte Ausführungsbeispiele des erfindungsgemäßen Verfahrens ergeben sich durch vorteilhafte Ausführungsformen des erfindungsgemäßen Instrumentariums.

Die nachfolgende Beschreibung bevorzugter Ausführungsformen der Erfindung dient im Zusammenhang mit der Zeichnung der näheren Erläuterung der Erfindung. Es werden vorteilhafte Ausführungsformen eines erfindungsgemäßen Instrumentariums beschrieben, mit denen das erfindungsgemäße Verfahren durchführbar ist. Es zeigen:
- Figur 1:: eine schematische perspektivische Teildarstellung eines erfindungsgemäßen Instrumentariums;
- Figur 2:: eine Verformungseinrichtung des Instrumentariums aus Figur 1;
- Figur 3:: eine schematische perspektivische Teildarstellung (mit Ausnahme einer Verformungseinrichtung) einer weiteren Ausführungsform des erfindungsgemäßen Instrumentariums; und
- Figur 4:: eine schematische perspektivische Darstellung eines Navigations-systems eines erfindungsgemäßen Instrumentariums.

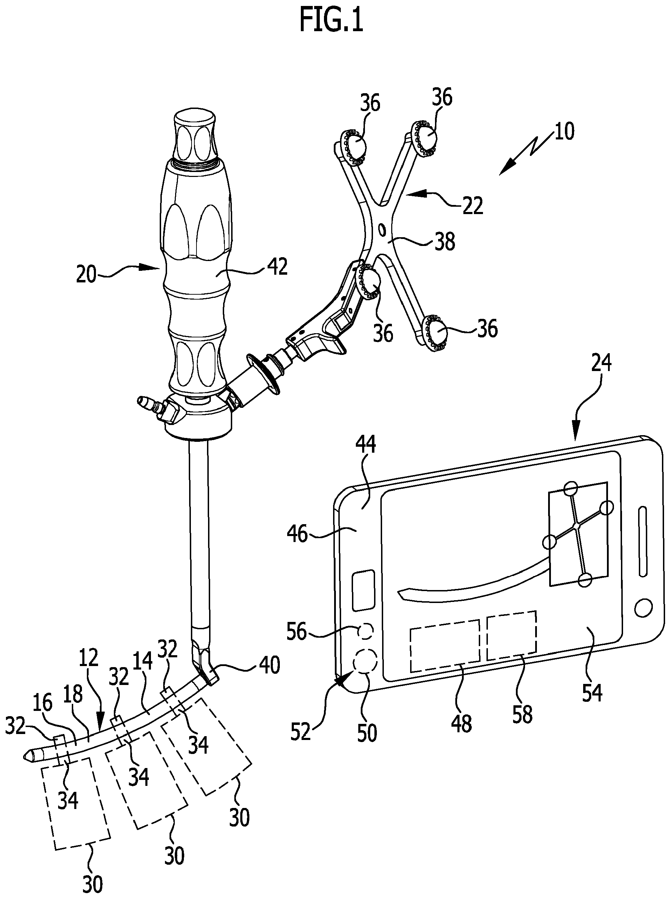

Figur 1 zeigt in perspektivischer Darstellung eine mit dem Bezugszeichen 10 belegte vorteilhafte Ausführungsform eines medizinischen Instrumentariums. Das Instrumentarium 10 umfasst einen medizintechnischen Artikel 12. Vorliegend handelt es sich bei dem Artikel 12 um ein Implantat 14, nämlich in Gestalt eines Stabilisierungselementes 16 eines chirurgischen Fixationssystems. Das Stabilisierungselement 16 ist als eine Längserstreckung aufweisender Stab 18 ausgestaltet.

Das Instrumentarium 10 umfasst ferner ein Implantationswerkzeug 20 für das Stabilisierungselement 16. Außerdem umfasst das Instrumentarium 10 eine medizinische Markiereinrichtung 22 und ein medizintechnisches Navigationssystem 24.

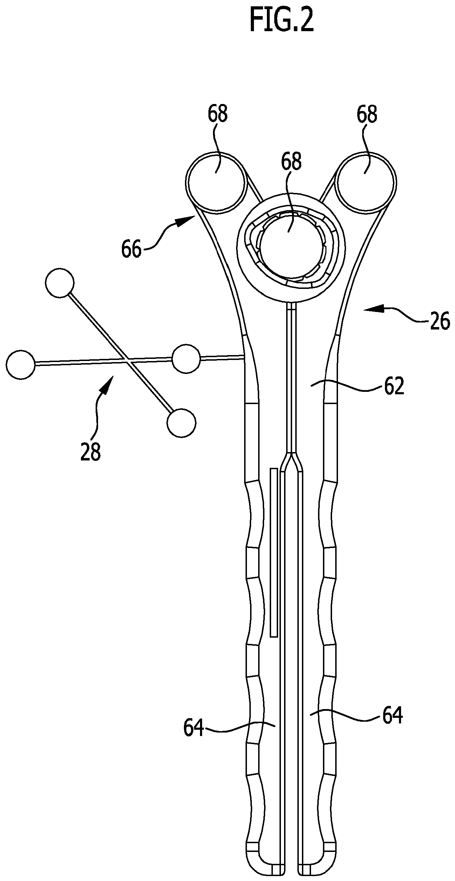

Ferner umfasst das Instrumentarium 10 eine in Figur 2 in Seitenansicht dargestellte Verformungseinrichtung 26 zum Verformen des Stabilisierungselementes 16 und eine daran angeordnete weitere medizinische Markiereinrichtung 28.

Das Fixationssystem dient zur Fixierung von in Figur 1 schematisch dargestellten Wirbelkörpern 30 gegen Bewegung relativ zueinander. Zu diesem Zweck weist das Fixationssystem Verankerungselemente 32 auf in Gestalt von Knochenschrauben 34, die an den Wirbelkörpern 30 fixiert sind. In Anwendung des Fixationssystems wird das Stabilisierungselement 16 an den Knochenschrauben 34 fixiert. Über das stabförmige Stabilisierungselement 16 sind die Knochenschrauben 34 starr miteinander verbindbar.

Um die erforderliche Relativposition der Knochenschrauben 34 und damit der Wirbelkörper 30 sicherzustellen, ist es in der Praxis von Bedeutung, dass der Stab 18 eine gewünschte, definierte Form aufweist. Außerdem ist es von Bedeutung, dass die Position des Stabes 18 im Hinblick auf die Führung des Implantationswerkzeuges 20 bekannt ist. Die Position und die Form des Stabes 18 werden anhand des Navigationssystems 24 wie nachfolgend erläutert ermittelt.

Die Markiereinrichtung 22 ist vorliegend als sogenannter Rigid Body ausgestaltet. Die Markiereinrichtung 22 umfasst eine Mehrzahl von Markierelementen 36. Die Markierelemente 36 sind an einer gemeinsamen Halterung 38 gehalten. Vorzugsweise sind die Markierelemente 36 retroreflektierend für insbesondere sichtbares Licht ausgebildet.

Die Markiereinrichtung 22 ist beim Instrumentarium 10 in definierter räumlicher Anordnung mittelbar am Stab 18 gehalten. Zu diesem Zweck dient das Implantationswerkzeug 20. Der Stab 18 ist beispielsweise an einem distalen Ende 40 des Implantationswerkzeugs 20 gehalten und nimmt relativ zu diesem eine definierte räumliche Anordnung ein. Die Markiereinrichtung 22 ist ebenfalls in definierter räumlicher Anordnung am Implantationswerkzeug 20 gehalten, beispielsweise an oder nahe einem Griffelement 42 des Implantationswerkzeugs 20.

Aus diesem Grund ist die räumliche Lage und Orientierung des Stabes 18 relativ zur Lage und Orientierung der Markiereinrichtung 22 bekannt. Wird die Markiereinrichtung 22 mittels des Navigationssystems 24 verfolgt, kann dadurch auch auf die Lage und die Orientierung des Stabes 18 geschlossen werden. Die Markiereinrichtung 22 definiert ein Referenzkoordinatensystem.

Zur erleichterten Handhabung und zur einfachen Ausgestaltung des Instrumentariums 10 ist das Navigationssystem 24 vorliegend als handhaltbares, integriertes Navigationssystem ausgestaltet. Beispielsweise handelt es sich dabei um ein(en) Smartphone 44 oder Tablet-Computer. "Integriert" ist vorliegend insbesondere dahingehend aufzufassen, dass die Komponenten des Navigationssystems 24 in einem gemeinsamen Gehäuse 46 des Navigationssystems 24 angeordnet sind. Beispielsweise weist das Navigationssystem 24 eine im Gehäuse 46 angeordnete Datenverarbeitungseinheit 48 auf.

Ferner ist eine eine Kamera 50 aufweisende optische Erfassungseinheit 52 im Gehäuse 46 angeordnet. Günstigerweise ist genau eine digitale Kamera 50 vorgesehen. Im Gehäuse 46 ist außerdem eine Hinweiseinheit angeordnet, ausgestaltet als Anzeigeeinheit 54. Die Anzeigeeinheit 54 ist insbesondere ein Touchscreen.

Ferner umfasst das Navigationssystem 24 eine im Gehäuse 46 angeordnete Beleuchtungseinheit 56, die insbesondere eine LED-Lichtquelle umfasst. Mit der Beleuchtungseinheit 56 kann insbesondere sichtbares Licht in Richtung der Markiereinrichtung 22 emittiert werden, und von deren Markierelementen 36 reflektiertes Licht kann von der Kamera 50 empfangen werden.

Die Datenverarbeitungseinheit 48 umfasst zum Beispiel einen Mikroprozessor oder ist als solcher ausgestaltet, auf dem ein Anwendungsprogramm des Navigationssystems 24 ausgeführt werden kann. Das Anwendungsprogramm umfasst insbesondere Algorithmen für die Bildverarbeitung.

Das Instrumentarium 10 kann ferner eine Speichereinheit 58 umfassen, die vorzugsweise im Gehäuse 46 des Navigationssystems 24 angeordnet ist. In der Speichereinheit 58 können Informationen über den Artikel 12 gespeichert sein, beispielsweise dessen Typ und insbesondere dessen Abmessungen wie Länge, Breite, Krümmung oder Durchmesser.

Beim Instrumentarium 10 kann mit dem Navigationssystem 24 die Form des Stabes 18 auf einfache Weise ermittelt werden. Zu diesem Zweck kann die Bedienperson mit der Kamera 50, wie in Figur 1 (ohne Bedienperson) schematisch dargestellt, Aufnahmen der Markiereinrichtung 22 und des Stabes 18 erstellen.

Die Datenverarbeitungseinheit 48 ist so programmiert, dass sie anhand einer Aufnahme, die von der Kamera 50 erstellt wird, die Position und insbesondere die Form des Stabes 18 ermittelt. Dies ist insbesondere dadurch möglich, dass aufgrund der bekannten räumlichen Anordnung der Markiereinrichtung 22 zum Stab 18 durch Lage und Orientierung der Markiereinrichtung 22 relativ zur Kamera 50, wie vorstehend erläutert, auch auf die Lage und Orientierung des Stabes 18 geschlossen werden kann. Die Algorithmen für die Bildverarbeitung, die im Navigationssystem 24 hinterlegt sind, analysieren die Aufnahme der Kamera 50.

Ergänzend können Informationen über den Artikel 12, die in der Speichereinheit 58 gespeichert sind, zur Analyse der Aufnahme herangezogen werden. Dies gibt beispielsweise die Möglichkeit, den Artikel 12 als Stab 18 zu identifizieren und dadurch dessen Form auf einfachere Weise festzustellen.

Vorzugsweise können der Bedienperson an der Anzeigeeinheit 54 Hinweise bereitgestellt werden, dass und wie, insbesondere in welcher Orientierung, die mindestens eine Aufnahme zu erstellen ist. Dabei ist es günstig, wenn bei einem eine Längserstreckung aufweisenden Artikel 12, wie dem Stab 18, eine optische Achse der Kamera 50 ungefähr quer zur Längserstreckung des Artikels 12 ausgerichtet ist.

Denkbar ist auch, dass die Bedienperson mittels der Kamera 50 zwei oder mehr Aufnahmen der Markiereinrichtung 22 und des Stabes 18 in vorzugsweise unterschiedlicher Orientierung erstellt. Dies erlaubt es der Datenverarbeitungseinheit 48, die Form und Position des Stabes 18 noch zuverlässiger zu ermitteln. Bei mehr als zwei Aufnahmen ist es von Vorteil, wenn die Aufnahmen und die jeweilige Orientierung des Navigationssystems 24 relativ zur Markiereinrichtung 22 und zum Stab 18 paarweise voneinander unterschiedlich sind.

An der Anzeigeeinheit 54 können die Aufnahmen selbst oder eine Darstellung des Stabes 18, ermittelt anhand der einen oder mehreren Aufnahmen, dargestellt werden. Dies ist in Figur 1 schematisch gezeigt.

Das Instrumentarium 10 erlaubt es insbesondere, die Form und Position des Stabes 18 präoperativ, ex-situ oder vorteilhafterweise auch in-situ zu ermitteln.

Ist die Form des Stabes 18 ermittelt, kann die Datenverarbeitungseinheit 48 feststellen, ob der Stab 18 die erforderliche Form und insbesondere Krümmung aufweist, damit die Knochenschrauben 34 wie vorgesehen miteinander verbunden werden können. Ist dies der Fall, kann der Stab 18 mit dem Implantationswerkzeug 20 implantiert werden.

Als besonders vorteilhaft erweist es sich dabei, dass die Markiereinrichtung 22 am Implantationswerkzeug 20 festgelegt ist. Unter Verfolgung der Markiereinrichtung 22 mit dem Navigationssystem 24 kann der Stab 18 bei der Implantation getrackt werden. Da die Form und die Position des Stabes 18 im Referenzkoordinatensystem bekannt sind, kann der Benutzer an der Anzeigeeinheit 54 mit Hinweisen zum Führen des Implantationswerkzeuges 20 angeleitet werden. Dabei wird davon ausgegangen, dass die Lage der Knochenschrauben 34 in dem durch die Markiereinrichtung 22 definierten Referenzkoordinatensystem bekannt ist. Beispielsweise ist eine der Knochenschrauben 34 mit einer in der Zeichnung nicht dargestellten Markiereinrichtung versehen und die Position der anderen Knochenschrauben 34 relativ zu dieser Markiereinrichtung bekannt. Dadurch kann der Stab 18 beim Einsetzen relativ zu den Knochenschrauben 34 getrackt werden.

Figur 2 zeigt eine Verformungseinrichtung 26, um die Form des Stabes 18 zu verändern. Die Verformungseinrichtung 26 ist ausgestaltet als Biegeeinrichtung 62, insbesondere als handhaltbare und handbetätigbare Biegezange. Die Biegeeinrichtung 62 weist beispielsweise relativ zueinander verschwenkbare Branchen 64 auf. An einem distalen Ende 66 der Branchen 64 können Anlageelemente 68 angeordnet sein zum Anlegen am Stab 18. Durch Handbetätigung kann der Stab 18 gebogen werden.

An der Biegeeinrichtung 62 ist vorzugsweise eine weitere Markiereinrichtung 28 angeordnet. Die Markiereinrichtung 28 kann entsprechend wie die Markiereinrichtung 22 vom Navigationssystem 24 verfolgt werden und stimmt in seiner Funktion mit dieser überein.

Wenn die Form des Stabes 18, die anhand der einen Aufnahme ermittelt worden ist, nicht mit der erforderlichen Form übereinstimmt, kann der Stab 18 durch Biegen mit der Biegeeinrichtung 62 in die gewünschte Form überführt werden. Zu diesem Zweck ist es zum Beispiel möglich, dass die Datenverarbeitungseinheit 48 an der Anzeigeeinheit 54 Hinweise für die Bedienperson bereitstellt, wie die Biegeeinrichtung 62 zu betätigen ist.

Das Biegen des Stabes 18 kann insbesondere in-situ erfolgen. Von Vorteil ist es, wenn die Biegeeinrichtung 62 dabei über die Markiereinrichtung 28 vom Navigationssystem 24 verfolgt wird. Dies gibt die Möglichkeit, die Bedienperson insbesondere in-situ zum Biegen des Stabes 18 anzuleiten. Ansatzpunkte der Anlageelemente 68 am Stab 18, die Biegeebene und der Biegeradius können durch Verfolgen der Biegeeinrichtung 62 relativ zum Stab 18 überprüft und verifiziert werden. Erforderlichenfalls können an der Anzeigeeinheit 54 Hinweise für die Bedienperson bereitgestellt werden, die Biegeeinrichtung 62 am Stab 18 zu repositionieren.

Anstelle der handbetätigten Verformungseinrichtung 26 kann, wie bereits erwähnt, auch eine maschinelle Verformungseinrichtung zum Einsatz kommen.

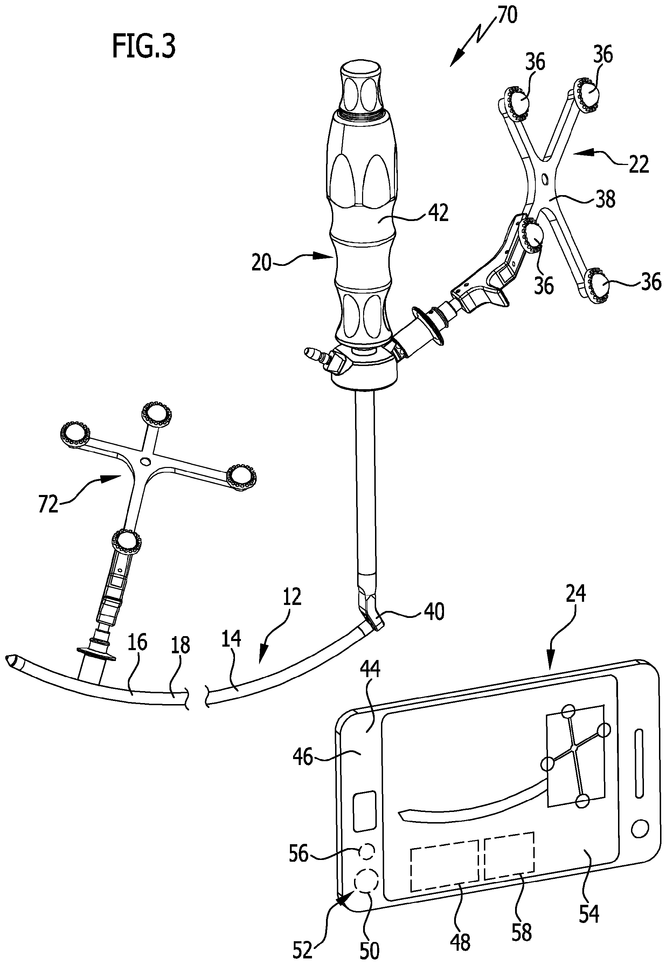

Die Figur 3 zeigt in einer der Figur 1 ähnlichen Weise eine mit dem Bezugszeichen 70 belegte zweite vorteilhafte Ausführungsform eines erfindungsgemäßen Instrumentariums. Die mit dem Instrumentarium 10 erzielbaren Vorteile können mit dem Instrumentarium 70 ebenfalls erzielt werden. Zur Vermeidung von Wiederholungen kann auf voranstehende Ausführungen verwiesen werden. Die Verformungseinrichtung 26 kann Bestandteil des Instrumentariums 70 sein.

Für gleiche oder gleichwirkende Merkmale und Bauteile der Instrumentarien 70 und 10 werden identische Bezugszeichen benutzt.

Beim Instrumentarium 70 ist der Stab 18 im Verhältnis zum Ausführungsbeispiel des Instrumentariums 10 länger ausgestaltet.

Außerdem weist das Instrumentarium 70 eine weitere Markiereinrichtung 72 auf, die entsprechend wie die Markiereinrichtung 22 vom Navigationssystem 24 verfolgt werden kann und mit dieser in seiner Funktion übereinstimmt. Die Markiereinrichtung 72 kann am Stab 18 an unterschiedlichen Positionen lösbar festgelegt werden, beispielsweise durch Verklemmung. Die Figur 3 zeigt schematisch eine Festlegung der Markiereinrichtung 72 im Bereich eines distalen Endabschnittes des Stabes 18.

Die Markiereinrichtung 72 ist auf diese Weise in definierter räumlicher Anordnung zum Stab 18 positioniert.

Mit der Kamera 50 können mehrere Aufnahmen erstellt werden, die jeweils mindestens eine Markiereinrichtung 22, 72 und einen Abschnitt des Stabes 18 erfassen. Beispielsweise wird ein Abschnitt des Stabes 18 mit der Markiereinrichtung 72 erfasst. Die Position und die Form dieses Abschnittes des Stabes 18 kann anhand einer jeweiligen Aufnahme von der Datenverarbeitungseinheit 48 ermittelt werden.

Dies gibt die Möglichkeit, die Form des Stabes 18 sukzessive durch mehrere Aufnahmen zu erfassen, wobei eine Referenzierung der Position des Stabes 18 insgesamt zusammen anhand der Markiereinrichtung 22 erfolgen kann.

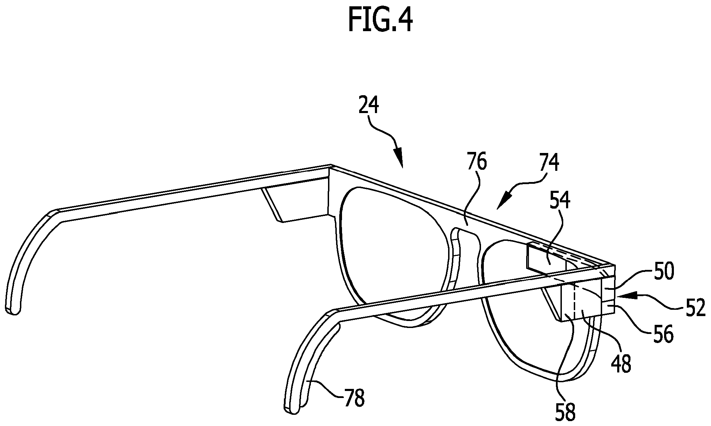

Figur 4 zeigt eine im Vergleich zum Smartphone 44 andersartige Ausgestaltung eines Navigationssystems 24, nämlich in Gestalt einer Datenbrille 74. Die Datenbrille 74 kann statt oder ergänzend zu dem Smartphone 44 bei einem erfindungsgemäßen Instrumentarium zum Einsatz kommen, d. h. insbesondere den Instrumentarien 10 und/oder 70.

Die Datenbrille 74 kann von einer in der Zeichnung nicht dargestellten Bedienperson nach Art einer üblichen Brille getragen werden, aufliegend auf den Ohren und auf der Nase. Es kann vorgesehen sein, dass Brillengläser am Brillengestell 76 eingesetzt sind, dies ist jedoch nicht zwingend erforderlich.

An der Datenbrille ist die die Kamera 50 aufweisende Erfassungseinheit 52 gehalten, die Datenverarbeitungseinheit 48, die Speichereinheit 58 sowie die Anzeigeeinheit 54. Entsprechend wie beim Smartphone 44 können der Bedienperson Bildinhalte an der Bildanzeige der Anzeigeeinheit 54 dargestellt werden.

Auch die Beleuchtungseinheit 56 ist am Brillengestell 76 gehalten. Zur Energieversorgung kann am Brillengestell 76 ferner eine Batterie 78 gehalten sein.

### Bezugszeichenliste:

- 10: Instrumentarium
- 12: Artikel
- 14: Implantat
- 16: Stabilisierungselement
- 18: Stab
- 20: Implantationswerkzeug
- 22: Markiereinrichtung
- 24: Navigationssystem
- 26: Verformungseinrichtung
- 28: Markiereinrichtung
- 30: Wirbelkörper
- 32: Verankerungselement
- 34: Knochenschraube
- 36: Markierelement
- 38: Halterung
- 40: distales Ende
- 42: Griffelement
- 44: Smartphone
- 46: Gehäuse
- 48: Datenverarbeitungseinheit
- 50: Kamera
- 52: Erfassungseinheit
- 54: Anzeigeeinheit
- 56: Beleuchtungseinheit
- 58: Speichereinheit
- 62: Biegeeinrichtung
- 64: Branche
- 66: distales Ende
- 68: Anlageelement
- 70: Instrumentarium
- 72: Markiereinrichtung
- 74: Datenbrille
- 76: Brillengestell
- 78: Batterie

## Patentansprüche

1. Medizinisches Instrumentarium mit einem handhaltbaren integrierten Navigationssystem (24), das, in einem gemeinsamen Gehäuse angeordnet, eine eine Kamera (50) aufweisende optische Erfassungseinheit (52) aufweist und eine mit dieser gekoppelte Datenverarbeitungseinheit (48), mit einem medizintechnischen Artikel (12) sowie einer Markiereinrichtung (22), welche direkt oder indirekt am Artikel (12) gehalten und in definierter räumlicher Anordnung zu diesem positioniert ist, oder welche Markiereinrichtung vom Artikel (12) umfasst oder gebildet ist, wobei mit dem Navigationssystem (24) die Lage und Orientierung der ein Referenzkoordinatensystem definierenden Markiereinrichtung (22) ermittelbar ist, wobei der Artikel (12) ein Implantat (14) in Gestalt eines Stabilisierungselementes (16) eines chirurgischen Fixationssystems ist, wobei mittels der Erfassungseinheit (52) mindestens eine Aufnahme der Markiereinrichtung (22) und des Stabilisierungselementes (16) erstellbar ist und die Position und Form des Stabilisierungselementes (16) von der Datenverarbeitungseinheit (48) anhand genau einer Aufnahme der mindestens einen Aufnahme ermittelbar ist, wobei in einer Speichereinheit (58) Informationen über das Stabilisierungselement (16) gespeichert sind, die von der Datenverarbeitungseinheit (48) bei der Ermittlung der Form des Stabilisierungselementes (16) herangezogen werden, und wobei die Position des Stabilisierungselementes (16) im Referenzkoordinatensystem anhand der definierten räumlichen Beziehung der Markiereinrichtung (22) zum Stabilisierungselement (16) feststellbar ist.

2. Instrumentarium nach Anspruch 1, **dadurch gekennzeichnet, dass** das Navigationssystem (24) ein Smartphone (44) oder ein Tablet-Computer ist.

3. Instrumentarium nach Anspruch 1, **dadurch gekennzeichnet, dass** das Navigationssystem (24) eine Datenbrille (74) ist oder umfasst.

4. Instrumentarium nach einem der voranstehenden Ansprüche, **dadurch gekennzeichnet, dass** das Navigationssystem (24) eine mit der Datenverarbeitungseinheit (48) gekoppelte Anzeigeeinheit (54) umfasst, an der die Aufnahmen des Stabilisierungselementes (16) darstellbar sind und/oder Hinweise für eine Bedienperson, die Aufnahmen durchzuführen und/oder eine Darstellung des Stabilisierungselementes (16), ermittelt anhand der Aufnahmen.

5. Instrumentarium nach einem der voranstehenden Ansprüche, **dadurch gekennzeichnet, dass** das Instrumentarium ein Implantationswerkzeug (20) für das Stabilisierungselement (16) aufweist, an dem dieses und die Markiereinrichtung (22) gehalten sind.

6. Instrumentarium nach Anspruch einem der voranstehenden Ansprüche, **dadurch gekennzeichnet, dass** das Stabilisierungselement (16) ein Stab (18) ist.

7. Instrumentarium nach einem der voranstehenden Ansprüche, **dadurch gekennzeichnet, dass** das Instrumentarium (10; 70) eine Verformungseinrichtung (26) aufweist, mit der die Form des Stabilisierungselementes (16) veränderbar ist.

8. Instrumentarium nach Anspruch 7, **dadurch gekennzeichnet, dass** die Verformungseinrichtung (26) eine Biegeeinrichtung (62) zum Biegen eines Stabes (18) ist, als der das Stabilisierungselement (16) ausgestaltet ist.

9. Instrumentarium nach einem der voranstehenden Ansprüche, **dadurch gekennzeichnet, dass** das Stabilisierungselement (16) Markierelemente der Markiereinrichtung umfasst oder bildet, beispielsweise ausgestaltet als Punkte oder Linien am Stabilisierungselement (16).

10. Instrumentarium nach einem der voranstehenden Ansprüche, **dadurch gekennzeichnet, dass** die Markiereinrichtung (22) am Stabilisierungselement (16) lösbar festgelegt oder festlegbar ist.

11. Instrumentarium nach einem der voranstehenden Ansprüche, **dadurch gekennzeichnet, dass** die Markiereinrichtung einstückig mit dem Stabilisierungselement (16) verbunden ist, vorzugsweise dass eine Sollbruchstelle vorgesehen ist zum Trennen der Markiereinrichtung vom Stabilisierungselement (16).

12. Instrumentarium nach einem der voranstehenden Ansprüche, **dadurch gekennzeichnet, dass** eine Mehrzahl von Markiereinrichtungen (22, 72) direkt oder indirekt am Stabilisierungselement (16) gehalten und in definierter räumlicher Anordnung zu diesem positioniert oder von diesem umfasst oder gebildet ist, wobei anhand von mehreren Aufnahmen, die jeweils mindestens eine Markiereinrichtung (22, 72) und einen Abschnitt des Stabilisierungselementes (16) erfassen, die Position und Form des Abschnittes des Stabilisierungselementes (16) von der Datenverarbeitungseinheit (48) ermittelbar ist.

13. Verfahren zum Ermitteln der Form eines medizintechnischen Artikels unter Einsatz eines Instrumentariums nach einem der voranstehenden Ansprüche, wobei der Artikel ein Implantat in Gestalt eines Stabilisierungselementes eines chirurgischen Fixationssystems ist, wobei mittels der Erfassungseinheit mindestens eine Aufnahme der Markiereinrichtung und des Stabilisierungselementes erstellt wird und die Position und Form des Stabilisierungselementes von der Datenverarbeitungseinheit anhand genau einer Aufnahme der mindestens einen Aufnahme ermittelt wird.

## Claims

1. Medical instrumentation with a hand-held, integrated navigation system (24), which, arranged in a common housing, comprises an optical detection unit (52) comprising a camera (50) and a data processing unit (48) coupled to the detection unit, with a medical article (12) and with a marking device (22) which is held directly or indirectly on the article (12) and is positioned in a defined spatial arrangement in relation thereto, or which marking device is comprised by or formed by the article (12), the location and orientation of the marking device (22), which defines a reference coordinate system, being determinable with the navigation system (24), the article (12) being an implant (14) in the form of a stabilization element (16) of a surgical fixation system, it being possible for at least one image of the marking device (22) and the stabilization element (16) to be taken by means of the detection unit (52), and for the position and shape of the stabilization element (16) to be determined by the data processing unit (48) on the basis of precisely one image of the at least one image, wherein information about the stabilization element (16), which is used by the data processing unit (48) when determining the shape of the stabilization element (16), is stored in a storage unit (58), and wherein the position of the stabilization element (16) can be established in the reference coordinate system owing to the defined spatial relationship of the marking device (22) to the stabilization element (16).

2. Instrumentation in accordance with claim 1, **characterized in that** the navigation system (24) is a smartphone (44) or a tablet computer.

3. Instrumentation in accordance with claim 1, **characterized in that** the navigation system (24) is or comprises smart glasses (74).

4. Instrumentation in accordance with any one of the preceding claims, **characterized in that** the navigation system (24) comprises a display unit (54) which is coupled to the data processing unit (48) and on which it is possible to show the images of stabilization element (16) and/or instructions for an operator for taking the images and/or a representation of the stabilization element (16), determined on the basis of the images.

5. Instrumentation in accordance with any one of the preceding claims, **characterized in that** the instrumentation comprises an implantation tool (20) for the stabilization element (16), on which the stabilization element (16) and the marking device (22) are held.

6. Instrumentation in accordance with any one of the preceding claims, **characterized in that** the stabilization element (16) is a rod (18).

7. Instrumentation in accordance with any one of the preceding claims, **characterized in that** the instrumentation (10; 70) comprises a reshaping device (26) with which the shape of the stabilization element (16) is changeable.

8. Instrumentation in accordance with claim 7, **characterized in that** the reshaping device (26 is a bending device (62) for bending a rod (18), as which the stabilization element (16) is configured.

9. Instrumentation in accordance with any one of the preceding claims, **characterized in that** the stabilization element (16) comprises or forms marking elements of the marking device, for example, configured as points or lines on the stabilization element (16).

10. Instrumentation in accordance with any one of the preceding claims, **characterized in that** the marking device (22) is releasably fixed or fixable on the stabilization element (16).

11. Instrumentation in accordance with any one of the preceding claims, **characterized in that** the marking device is connected in one piece to the stabilization element (16), preferably **in that** a predetermined breaking point is provided for separating the marking device from the stabilization element (16).

12. Instrumentation in accordance with any one of the preceding claims, **characterized in that** a plurality of marking devices (22, 72) are held directly or indirectly on the stabilization element (16) and are positioned in a defined spatial arrangement in relation to the stabilization element (16) or are comprised by or formed by the stabilization element (16), it being possible on the basis of several images which each record at least one marking device (22, 72) and a section of the stabilization element (16) for the position and shape of the section of the stabilization element (16) to be determined by the data processing unit (48).

13. Method for determining the shape of a medical article using an instrumentation in accordance with any one of the preceding claims, wherein the article is an implant in the form of a stabilization element of a surgical fixation system, wherein at least one image of the marking device and the stabilization element is taken by means of the detection unit, and the position and shape of the stabilization element are determined by the data processing unit on the basis of precisely one image of the at least one image.

## Revendications

1. Ensemble d'instruments médicaux avec un système de navigation intégré (24) pouvant être tenu à la main qui, disposé dans un boîtier commun, comporte une unité d'acquisition optique (52) comportant un appareil photographique (50) et une unité de traitement de données (48) couplée à celle-ci, avec un article médico-technique (12) ainsi qu'un dispositif de marquage (22), qui est maintenu directement ou indirectement sur l'article (12) et positionné dans une disposition spatiale définie par rapport à celui-ci, ou lequel dispositif de marquage est compris ou formé par l'article (12), où avec le système de navigation (24), la position et l'orientation du dispositif de marquage (22) qui définit un système de coordonnées de référence, peuvent être déterminées, où l'article (12) est un implant (14) sous la forme d'un élément de stabilisation (16) d'un système de fixation chirurgical, où au moyen de l'unité d'acquisition (52) au moins une prise de vue du dispositif de marquage (22) et de l'élément de stabilisation (16) peut être créée et la position et la forme de l'élément de stabilisation (16) peuvent être déterminées par l'unité de traitement de données (48) à l'aide de précisément une prise de vue de la au moins une prise de vue, où des informations concernant l'élément de stabilisation (16) sont stockées dans une unité de stockage (58), qui sont utilisées par l'unité de traitement de données (48) pour déterminer la forme de l'élément de stabilisation (16), et où la position de l'élément de stabilisation (16) dans le système de coordonnées de référence peut être déterminée à l'aide de la relation spatiale définie entre le dispositif de marquage (22) et l'élément de stabilisation (16).

2. Ensemble d'instruments selon la revendication 1, **caractérisé en ce que** le système de navigation (24) est un smartphone (44) ou une tablette informatique.

3. Ensemble d'instruments selon la revendication 1, **caractérisé en ce que** le système de navigation (24) est ou comprend des lunettes de données (74).

4. Ensemble d'instruments selon l'une des revendications précédentes, **caractérisé en ce que** le système de navigation (24) comprend une unité d'affichage (54) couplée à l'unité de traitement de données (48), sur laquelle les prises de vue de l'élément de stabilisation (16) peuvent être représentées et/ou des indications pour un opérateur pour réaliser les prises de vue et/ou une représentation de l'élément de stabilisation (16), déterminée à l'aide des prises de vue.

5. Ensemble d'instruments selon l'une des revendications précédentes, **caractérisé en ce que** l'ensemble d'instruments comporte un outil d'implantation (20) pour l'élément de stabilisation (16), sur lequel celui-ci et le dispositif de marquage (22) sont maintenus.

6. Ensemble d'instruments selon l'une des revendications précédentes, **caractérisé en ce que** l'élément de stabilisation (16) est une tige (18).

7. Ensemble d'instruments selon l'une des revendications précédentes, **caractérisé en ce que** l'ensemble d'instruments (10; 70) comporte un dispositif de déformation (26) avec lequel la forme de l'élément de stabilisation (16) peut être modifiée.

8. Ensemble d'instruments selon la revendication 7, **caractérisé en ce que** le dispositif de déformation (26) est un dispositif de cintrage (62) pour cintrer une tige (18) sous forme de laquelle l'élément de stabilisation (16) est conçu.

9. Ensemble d'instruments selon l'une des revendications précédentes, **caractérisé en ce que** l'élément de stabilisation (16) comprend ou forme des éléments de marquage du dispositif de marquage, par exemple conçus sous forme de points ou de lignes sur l'élément de stabilisation (16).

10. Ensemble d'instruments selon l'une des revendications précédentes, **caractérisé en ce que** le dispositif de marquage (22) est fixé ou peut être fixé de manière amovible sur l'élément de stabilisation (16).

11. Ensemble d'instruments selon l'une des revendications précédentes, **caractérisé en ce que** le dispositif de marquage est relié d'une pièce à l'élément de stabilisation (16), de préférence **en ce qu'**un point destiné à la rupture est prévu pour la séparation du dispositif de marquage d'avec l'élément de stabilisation (16).

12. Ensemble d'instruments selon l'une des revendications précédentes, **caractérisé en ce qu'**une pluralité de dispositifs de marquage (22, 72) sont maintenus directement ou indirectement sur l'élément de stabilisation (16) et sont positionnés dans une disposition spatiale définie par rapport à celui-ci ou sont compris ou formés par celui-ci, où à l'aide de plusieurs prises de vue, qui acquièrent à chaque fois au moins un dispositif de marquage (22, 72) et une section de l'élément de stabilisation (16), la position et la forme de la section de l'élément de stabilisation (16) peuvent être déterminées par l'unité de traitement de données (48).

13. Procédé pour déterminer la forme d'un article médico-technique à l'aide d'un ensemble d'instruments selon l'une des revendications précédentes, où l'article est un implant sous la forme d'un élément de stabilisation d'un système de fixation chirurgical, où au moyen de l'unité d'acquisition au moins une prise de vue du dispositif de marquage et de l'élément de stabilisation est créée et la position et la forme de l'élément de stabilisation sont déterminées par l'unité de traitement de données à l'aide de précisément une prise de vue de la au moins une prise de vue.
